# EUROPEAN PATENT SPECIFICATION

(11) **EP 2 301 501 B1**
(45) Date of publication and mention of the grant of the patent: **01.04.2015**
(21) Application number: 09797809.2
(22) Date of filing: 30.06.2009
(51) Int. Cl.: A61F 13/15, A61F 13/49, A61F 13/56

(54) **METHOD OF MAKING WEARING ARTICLE AND THIS WEARING ARTICLE**
HERSTELLUNGSVERFAHREN FÜR EIN KLEIDUNGSSTÜCK UND KLEIDUNGSSTÜCK
PROCÉDÉ DE FABRICATION D'UN ARTICLE À PORTER ET ARTICLE À PORTER

(30) Priority: 15.07.2008 JP 2008184241; 03.06.2009 JP 2009134491
(43) Date of publication of application: 30.03.2011
(73) Proprietor: Unicharm Corporation, Ehime 799-0111 (JP)
(72) Inventor: KINOSHITA, Akiyoshi, Kanonji-shi Kagawa 769-1602 (JP); UKEGAWA, Kazuo, Kanonji-shi Kagawa 769-1602 (JP); MYOJO, Ryota, Kanonji-shi Kagawa 769-1602 (JP); AOYAGI, Natsuko, Kanonji-shi Kagawa 769-1602 (JP); TANAKA, Kayoko, Kanonji-shi Kagawa 769-1602 (JP); KENMOCHI, Yasuhiko, Kanonji-shi Kagawa 769-1602 (JP)
(74) Representative: Murgatroyd, Susan Elizabeth
(86) International application number: PCT/JP2009/061954
(87) International publication number: WO 2010/007881

(56) References cited:
- JP-A- 2006 087 568
- JP-A- 2006 150 068
- JP-A- 2006 175 007
- JP-A- 2008 012 115
- US-A1- 2004 006 327
- US-A1- 2005 113 793
- US-A1- 2008 009 816

## Description

### TECHNICAL FIELD

The present invention relates to a method of making a wearing article and this wearing article, more particularly to a wearing article such as a disposable diaper, toilet-training pants, incontinence briefs, sanitary shorts or a diaper cover and the method for making the same.

### RELATED ART

Disposable diapers having front and rear waist regions detachably fastened along the respective lateral zones thereof are well known, for example, from the disclosure of Japanese Patent Application Laid-Open Publication No. 2008-12115 (PATENT DOCUMENT 1). According to this PATENT DOCUMENT 1, the diaper comprises the chassis having the front and rear waist region, the crotch region, the side facing the wearer's skin and the side facing the wearer's clothes, and the hook and loop elements respectively formed on the front and rear waist regions along the lateral zones thereof. In the course of making the diaper, each of the mount members is folded in Z-shape or inverted Z-shape so that the fixed end defined by one of its lateral zones extending in the longitudinal direction may be fixed to the associated lateral zone of the chassis and the free end defined by the other of its lateral zones may extend outward in the transverse direction.

[PATENT DOCUMENT 1] JP2008-12115A corresponding to US2008/009816A1.

US2005/113793A1 and US2004/006327A1 each disclose a pants-type absorbent sanitary product of similar construction to the diaper disclosed in patent document 1.

### DISCLOSURE OF THE INVENTION

### PROBLEM TO BE SOLVED BY THE INVENTION

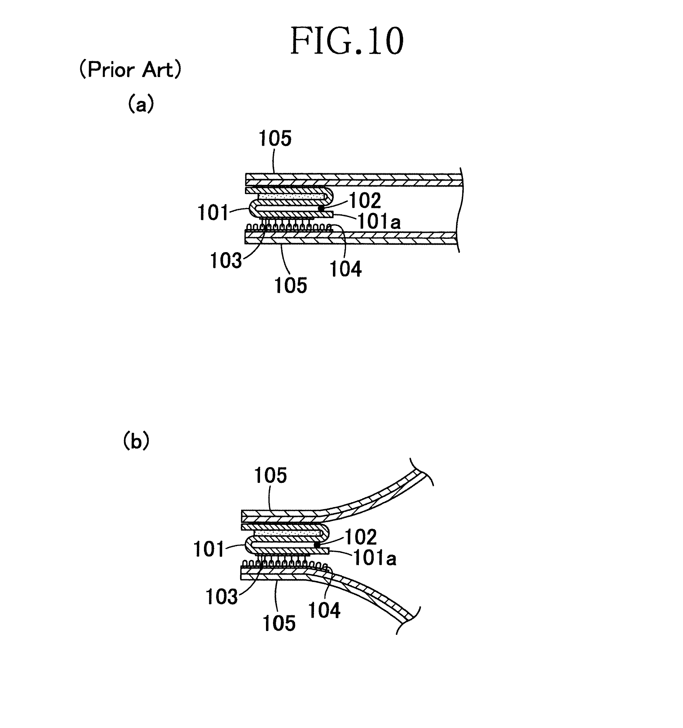

As illustrated by FIG. 10(a), mount member 101 is temporarily bonded by the associated adhesion spots 102 in the folded state to prevent the folded mount member 101 from being unfolded in untimely moment. These adhesion spots 102 are formed between two adjacent layers of the folded mount member 101 inside the free end 101a of the mount member 101 to further restrict the possibility that the folded mount member 101 might be unfolded in untimely moment. The mount member 101 temporarily bonded in this manner is provided with the hook elements 103 bonded thereto and the loop elements 104 are in engagement with these hook elements 103. To release the mount member 101 from the temporarily fixing effect of the adhesion spots 102, the chassis 105 may be opened to draw apart the front and rear waist regions from each other. However, as will be apparent from FIG. 10(b), the adhesion spots 102 are apt to follow the chassis 105 together with the mount member 101 and sometimes it may be impossible to release the mount member 101 from the temporarily fixing effect of the adhesion spots 102. If the chassis 105 is further opened to release the mount member 101 from the temporarily fixing effect of the adhesion spots 102, there is possibility that the hook elements are disengaged from the loop elements before the mount member 101 is released from the temporarily fixing effect of the adhesion spots 102. To avoid such situation in which the adhesion spots 102 follow movement of the chassis 105 together with the mount member 101, the free end 101a of the mount member 101 must be held with the fingers while the chassis 105 is opened. Consequentially, appreciably bothersome handling is required to release the mount member 101 from the temporarily fixing effect of the adhesion spots 102.

In view of the problem as has been described above, it is a principal object of the present invention to provide a method of making a wearing article and this wearing article improved so as to facilitate the mount members temporarily bonded by the arrays of adhesion spots in the folded states thereof to be unfolded when it is desired.

### MEASURE TO SOLVE THE PROBLEM

The present invention includes first, second and third aspects.

The object set forth above is achieved, according to the invention on the first and second aspects thereof, by an improvement in the method of making a wearing article comprising a chassis and mechanical fasteners as basic components, the chassis having a longitudinal direction, a transverse direction, a side facing wearer's skin and a side facing wearer's clothes, a first waist region corresponding to one of front and rear waist regions, a second waist region corresponding to the other of the front and rear waist regions and a crotch region extending between the first and second waist regions, and the mechanical fasteners include first mechanical fastener members provided along a pair of first lateral zones of the first waist region opposed to each other in the transverse direction so as to extend in the longitudinal direction and second mechanical fastener members provided along a pair of second lateral zones of the second waist region opposed to each other in the transverse direction so as to extend in the longitudinal direction wherein the first mechanical fastener members are detachably engaged with the second mechanical fastener members and wherein the first mechanical fastener members are formed along the first lateral zones by the intermediary of mount members.

The improvement according to the present invention on the first aspect thereof comprises steps of folding back each of the mount members along a first folding line extending in the longitudinal direction to define a first region and a second region, then along a second folding line also extending in the longitudinal direction to define the second region and a third region so that the second region may be lapped on the first region while the third region may be lapped on the second region, forming an array of adhesive spots between the second region and the third region for temporarily fixing the second region to the third region, bonding the third region to the associated first mechanical fastener member, engaging the first mechanical fastener member with the associated second mechanical fastener member, bonding the first region to the associated first lateral zone, and bonding the second mechanical fastener member to the associated second lateral zone, characterized in that the first mechanical fastener members are respectively divided into upper halves and lower halves in the longitudinal direction so as to form spaces therebetween; the arrays of adhesive spots are formed substantially over a full length of said mount member in said longitudinal direction; and the arrays of adhesive spots are formed in regions overlapping with the upper and lower halves of the first mechanical fastener members and in regions overlapping with said spaces.

The improvement according to the present invention on the second aspect thereof comprises steps of folding back each of the mount members along a first folding line extending in the longitudinal direction to define a first region and a second region, then along a second folding line also extending in the longitudinal direction to define the second region and a third region so that the second region may be lapped on the first region while the third region may be lapped on the second region, forming an array of adhesive spots between the second region and the third region for temporarily fixing the second region to the third region engaging the first mechanical fastener member with the associated second mechanical fastener member, bonding the third region to the associated first mechanical fastener member engaged with the second mechanical fastener member, bonding the first region to the associated first lateral zone, and bonding the second mechanical fastener member to the associated second lateral zone, characterized in that the first mechanical fastener members are respectively divided into upper halves and lower halves in the longitudinal direction so as to form spaces therebetween; the arrays of adhesive spots are formed substantially over a full length of said mount member in said longitudinal direction; and the arrays of adhesive spots are formed in regions overlapping with the upper and lower halves of the first mechanical fastener members and in regions overlapping with said spaces.

According to a preferred embodiment of the present invention on the first and second aspects thereof, a process of making the wearing article has a machine direction and a cross direction orthogonal to the machine direction and the chassis of the wearing article is continuously formed so that the transverse direction of the chassis corresponds to the machine direction, wherein the method further includes steps of feeding web forming the chassis in the machine direction, and cutting the chassis in the cross direction after the first mechanical fastener member has been engaged with the associated second mechanical fastener member, wherein the first lateral zones are formed along one end of the web extending in the machine direction and the second lateral zones are formed along the opposite end.

According to another preferred embodiment of the present invention on the first and second aspects thereof, respective pairs of the mount members of the wearing articles continuously formed in the machine direction are contiguous to each other in the respective third regions and cut off in these contiguous third regions in the cross direction.

The object set forth above is achieved, according to the present invention on the third aspect thereof, by an improvement in the wearing article comprising a chassis and mechanical fasteners as basic components, the chassis having a longitudinal direction, a transverse direction, a side facing wearer's skin and a side facing wearer's clothes, a first waist region corresponding to one of front and rear waist regions, a second waist region corresponding to the other of the front and rear waist regions and a crotch region extending between the first and second waist regions, and the mechanical fasteners include first mechanical fastener members provided along a pair of first lateral zones of the first waist region opposed to each other in the transverse direction so as to extend in the longitudinal direction and second mechanical fastener members provided along a pair of second lateral zones of the second waist region opposed to each other in the transverse direction so as to extend in the longitudinal direction wherein the first mechanical fastener members are detachably engaged with the second mechanical fastener members and wherein the first mechanical fastener members are formed along the first lateral zones by the intermediary of mount members.

In the improvement according to the present invention on the third aspect thereof, each of the mount members comprises a first folding line, a second folding line, a first region and a second region defined by the first folding line and the second folding line, and a third region defined together with the second regions by the second folding line wherein the second region is lapped on the first region and the third region is lapped on the second region, the third region is bonded to the associated first mechanical fastener member, the first region is bonded to the associated first lateral zone, and an array of adhesive spots is formed between the second region and the third region for temporarily fixing the second region to the third region, characterized in that the first mechanical fastener members are respectively divided into upper halves and lower halves in the longitudinal direction so as to form spaces therebetween; the arrays of adhesive spots are formed substantially over a full length of said mount member in said longitudinal direction; and the arrays of adhesive spots are formed in regions overlapping with the upper and lower halves of the first mechanical fastener members and in regions overlapping with said spaces.

According to one embodiment of the present invention on the third aspect thereof, the array of adhesive spots is formed so as to lap on a region in which the first mechanical fastener member is engaged with the associated second mechanical fastener member.

According to another embodiment of the present invention on the third aspect thereof, the array of adhesive spots is formed at least along the transversely inner end of the region in which the first mechanical fastener member is engaged with the associated second mechanical fastener member.

According to still another embodiment of the present invention on the third aspect thereof, the array of adhesive spots is intermittently formed substantially over a full length of the mount member in the longitudinal direction.

According to yet another embodiment of the present invention on the third aspect thereof, a plurality of the arrays of adhesive spots are formed so as to be spaced one from another in the transverse direction.

According to further another embodiment of the present invention on the third aspect thereof, the arrays of adhesive spots are arranged so that respective pairs of the individual adhesive spots in the plurality of arrays being adjacent in the transverse direction never lap on each other.

### EFFECT OF THE INVENTION

According to the present invention on the first, second and third aspects thereof, the array of adhesive spots formed between the second region and the third region of the folded mount member serves to prevent the folded mount member from being unfolded in untimely moment in the course of making the wearing article and thereby to restrict occurrence of any trouble. The mount member is provided so as to overlap the associated first mechanical fastener member, thereby facilitating the temporarily fixing effect to be eliminated without the possibility that the mount member might follow movement of the chassis.

In addition, according to the invention on the first aspect thereof, the second mechanical fastener member is bonded to the associated second lateral zone of the chassis after the first mechanical fastener member has been engaged with the associated second mechanical fastener member. In this way, no particular operation of alignment is necessary for engagement between the first and second mechanical fastener members and a production cost can be correspondingly reduced.

The wearing articles may be continuously formed in the machine direction and the continuously formed wearing articles in the form of the web may be successively cut off in the cross direction after the first mechanical fastener member has been engaged with the associated second mechanical fastener member. Such process well supports the wearing articles to be produced in large quantities.

In each of the wearing articles continuously formed in the machine direction, each pair of the adjacent mount members is cut off in the contiguous third regions in the cross direction. In this way, the mount member and the chassis can be cut at once and thereby the production cost can be correspondingly reduced.

The array of adhesive spots is formed so as to overlap the region in which the first mechanical fastener member is engaged with the associated second mechanical fastener member without extending outward beyond the region of the engagement both in the longitudinal direction and in the transverse direction. Consequentially, one surface of this array of adhesive spots is pulled by the first mechanical fastener member while the other surface is pulled by the second mechanical fastener member when it is tried to eliminate the temporarily fixing effect of the array of adhesive spots. Thus, it is possible further reliably to restrict the possibility that the mount member might follow the movement of the chassis.

By forming the array of adhesive spots adjacent the transversely inner side edge of the region in which the first mechanical fastener member is engaged with the associated second mechanical fastener member, the portion most apt to be peeled in the course of production can be temporarily fixed.

By intermittently forming the array of adhesive spots substantially over full length of the mount member in the longitudinal direction, there is substantially no apprehension that stiffness of the chassis might be unacceptably increased due to formation of the array of adhesive spots and the wearer might experience uncomfortable feeling due to such stiffness.

A plurality of the arrays of adhesive spots spaced in the transverse direction one from another allows the mount member to be temporarily fixed over a larger range and in more reliable manner. Furthermore, even if any one array of adhesive spots is unintentionally broken, the remaining array can maintain the desired function.

### BRIEF DESCRIPTION OF THE DRAWINGS

Fig. 1 is a perspective view of the diaper.
Fig. 2 is a plan view showing the diaper of Fig. 1 as been flatly developed.
Fig. 3 is a perspective view showing the diaper before it is practically used.
FIG. 4 (a) shows a partially cutaway sectional view taken along the line IV-IV in Fig. 3 and FIG. 4(b) shows a corresponding sectional view after the first arrays of adhesion spots have been broken.
Fig. 5 is a diagram schematically illustrating a process of making the diaper.
Fig. 6 is a diagram partially illustrating the process of making the diaper.
Fig. 7 is a diagram illustrating the first arrays of adhesion spots.
Fig. 8 is a view similar to FIG. 4(a), showing another embodiment.
Fig. 9 is a diagram similar to Fig. 6, illustrating another embodiment.
Fig. 10 illustrates the prior art.

### IDENTIFICATION OF REFERENCE NUMERALS USED IN THE DRAWINGS

- 1: diaper
- 2: chassis
- 3: mechanical fasteners
- 8: front waist region
- 9: rear waist region
- 10: crotch region
- 11: first lateral zones
- 12: second lateral zones
- 13, 14: mount members
- 15, 16: hook elements
- 17, 18: loop elements
- 26, 27: first regions
- 28, 29: second regions
- 30, 31: third regions
- 32, 33: first arrays of adhesion spots
- 35: first folding line
- 36: second folding line
- 41: first web
- 42: second web
- 44: cutting line
- 45: one end
- 46: opposite end
- 51, 52: second arrays of adhesion spots

### DETAILED DESCRIPTION OF THE PREFERRED EMBODIMENTS

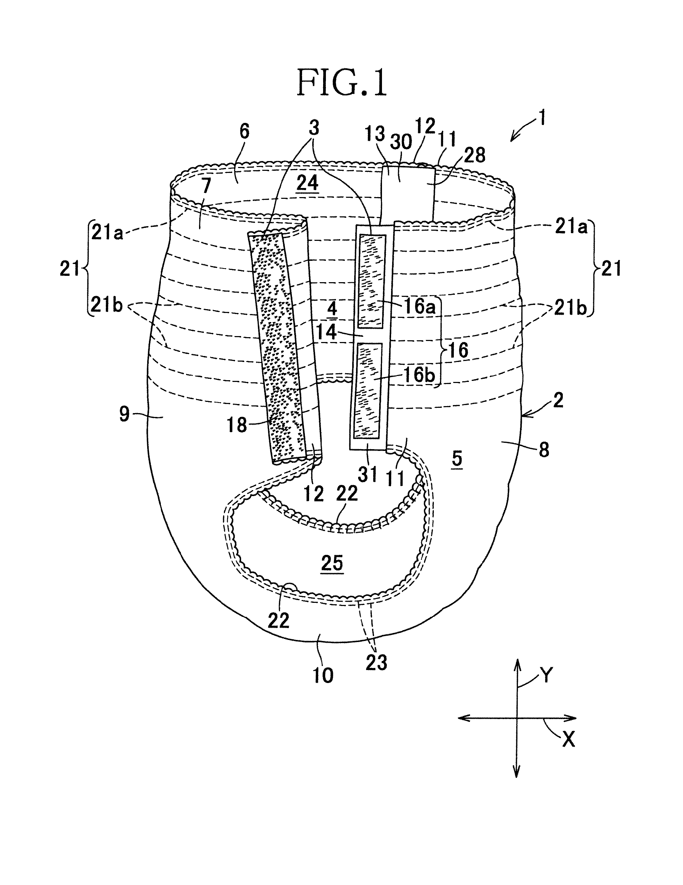

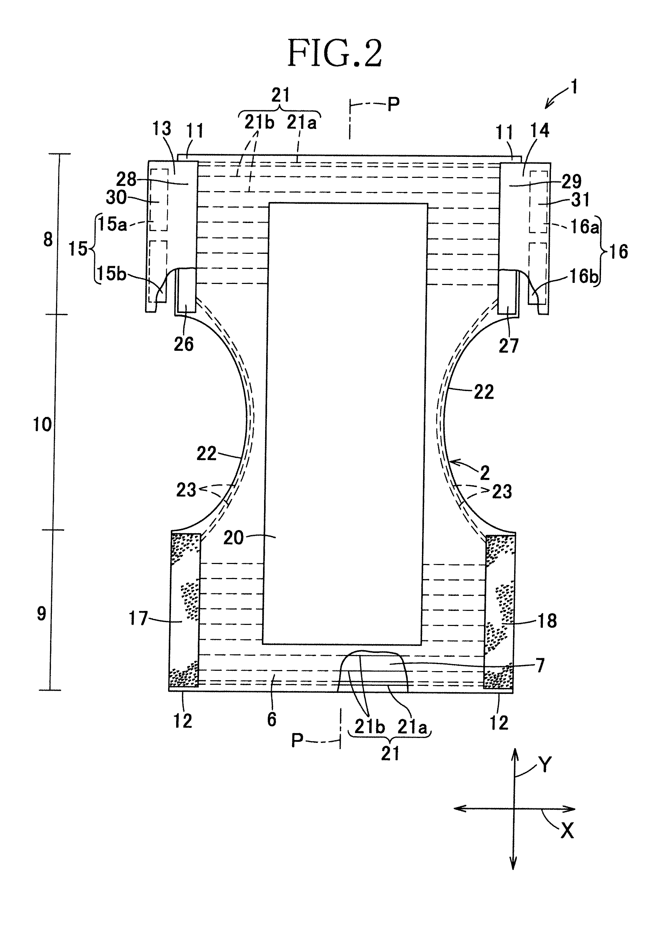

Figs. 1 through 6 illustrate one embodiment of the present invention in the form of an adult diaper as a typical embodiment of the invention. Fig. 1 is a perspective view of the diaper 1 as put on the wearer's body with one of transversely opposite lateral zones opened and Fig. 2 is a developed plan view of the diaper 1 partially broken away for convenience of illustration. As illustrated, the diaper 1 comprises a liquid-absorbent chassis 2 and mechanical fasteners 3 as basic components. The chassis 2 has a longitudinal direction Y, a transverse direction X, an inner side 4 facing the wearer's skin and an outer side facing the wearer' clothes. With respect to the plane defined by a sheet of Fig. 2, the near side corresponds to the inner side facing the wearer's skin and the remote side corresponds to the outer side facing the wearer's clothes. The chassis 2 includes a top-sheet 6 defining the inner side facing the wearer's skin, a back-sheet 7 defining the outer side 5 facing the wearer's clothes and a liquid-absorbent core 20 provided on the top-sheet 6 so as to face the wearer's skin more directly than the top-sheet 6. The top- and back-sheets 6, 7 may be formed, for example, by liquid-pervious fibrous non-woven fabric and the liquid-absorbent core 20 may be formed, for example, by a mixture of fluff pulp and super-absorbent polymer particles, all of them being the materials commonly used in the relevant technical field. Such chassis 2 comprises a front waist region 8, a rear waist region 9 and a crotch region 10 extending between the front and rear waist regions 8, 9.

The front waist region 8 has a pair of first lateral zones 11 opposed to each other in the transverse direction X so as to extending in the longitudinal direction Y and the rear waist region 9 has a pair of second lateral zones 12 opposed to each other in the transverse direction X so as to extending in the longitudinal direction Y. The top-sheet 6 is provided along the first lateral zones 11 with a pair of mount members (ear-like members) 13, 14 which are elongate in the longitudinal direction Y. Hook elements 15, 16 serving as first mechanical fastener members are attached to the chassis 2 by the intermediary of these mount members 13, 14.

The mount members 13, 14 may be formed, for example, by fibrous non-woven fabric and respectively comprise first regions 26, 27 boned to the top-sheet 6 along the respective first lateral zones 11, second regions 28, 29 folded back from the first regions 26, 27 toward the inner side facing the wearer's skin onto the first regions 26, 27, and third regions 30, 31 extending outward from the second regions 28, 29 in the transverse direction X.

The hook elements 15, 16 are bonded to the outer side facing the wearer's clothes in the third regions 30, 31 of the mount members 13, 14 extending outward. These hook elements 15, 16 are respectively divided in the longitudinal direction Y into upper halves 15a, 16a and lower halves 15b, 16b so as to be spaced from one another.

The top-sheet 6 is provided along the respective second lateral zones 12 with a pair of loop elements 17, 18 serving as second mechanical fastener members. The loop elements 17, 18 are dimensioned to have a substantially same length as a length of the second lateral zones 12 of the rear waist region 9 as measured in the longitudinal direction Y. In other words, these loop elements 17, 18 extend substantially along full length of the second lateral zones 12 in the longitudinal direction Y. The hook elements 15, 16 are detachably engaged with the associated loop elements 17, 18. As these hook elements 15, 16 and the loop elements 17, 18, "Velcro"^{™} or "Scotch Magic tape "^{™} commonly used in the relevant technical field may be used.

The front and rear waist regions 8, 9 are provided with waist-surrounding elastic members 21 attached thereto under tension but in contractible manner so as to extend in the transverse direction X. Specifically, these waist-surrounding elastic members 21 comprise first elastic members 21a extending along a peripheral edge of a waist-opening 24 and second elastic members 21b extending in the transverse direction X between the first elastic members 21a and the crotch region 10. These waist-surrounding elastic members 21 are sandwiched between the top- and back-sheets 6 and bonded to at least one of the top- and back-sheets 6, 7. The waist-surrounding elastic members 21 may be formed by a plurality of strings, strands or tape-segments made of rubber wherein the second elastic members 21b are arranged substantially at regular intervals in the longitudinal direction Y so as to be attached to the front and rear waist regions 6, 7 substantially over entire areas of these two waist regions 6, 7 and thereby to elasticize the front and rear waist regions 6, 7 in the transverse direction X. By elasticizing the front and rear waist regions 6, 7, desired fitness of the front and rear waist regions 6, 7 to the wearer's body is assured not only to prevent leak of body fluids such as urine but also to improve the appearance of the diaper 1 put on the wearer's body.

The waist-surrounding elastic members 21 in the front waist region 8 extend to respective inner sides of the mount members 13, 14 as viewed in the transverse direction X so that these elastic members 21 do not intersect with the respective mount members 13, 14. The hook elements 15, 16 are provided in third regions 30, 31 of the mount members 13, 14 extending outward from the second regions 28, 29 in the transverse direction X so that the waist-surrounding elastic members 21 do not overlap the hook elements 15, 16. Also in the rear waist region 9, the waist-surrounding elements 21 extend to the respective inner sides of the loop elements 17, 18 as viewed in the transverse direction X and do not overlap the loop elements 17, 18. Such arrangement that neither the hook elements 15, 16 nor the loop elements 17, 18 are overlapped by the waist-surrounding elastic members 21 is effective to restrict the hook elements 15, 16 and the loop elements 17, 18 from getting wrinkled under contraction of the waist-surrounding elastic members 21. Consequentially, there is substantially no apprehension that an area for engagement might be reduced due to getting wrinkles and thereby the engagement might become unstable. Thus it is assured that the hook elements 15, 16 are firmly engaged with the associated loop elements 17, 18.

Between the first and second lateral zones 11, 12 in the longitudinal direction Y, peripheral edges 22 of respective leg-openings 25 are defined by opposite lateral edges of the crotch region 10 so as to be curved toward a longitudinal center line P-P bisecting a dimension of the diaper 1 in the transverse direction X. The diaper 1 is provided along the respective peripheral edges 22 with leg-surrounding elastic members 23 attached thereto under tension. More specifically, these leg-surrounding elastic members 23 are sandwiched between the top- and back-sheets 6, 7 and bonded or joined to at least one of these top- and back-sheets 6, 7. The leg-surrounding elastic members 23 are formed by a plurality of strings, strands or tape-segments made of rubber so that contractile force of the rubber forming these elastic members 23 may bias the lateral edges 22 of the leg-openings 25 to fit around the wearer's thighs and thereby to prevent leak of body fluids such as urine.

The waist-surrounding elastic members 21 as well as the leg-surrounding elastic members 23 may be made of natural rubber or synthetic rubber such as polyurethane and, if this is convenient, these elastic members may be replaced by elasticized non-woven fibrous fabric or elastic plastic sheet, all of these stock materials for the elastic members being commonly used in the relevant technical field.

In the diaper 1 constructed as has been described above, the hook elements 15, 16 may be placed in engagement with the associated loop elements 17, 18 to join the front and rear waist regions 8, 9 to each other and thereupon to form the waist-opening 24 and a pair of the leg-openings 25. In this way, the diaper 1 is shaped into pull-on pants. To put such diaper 1 on the wearer's body, after the front and rear waist regions 8, 9 have been joined to each other, the wearer's legs may be inserted through the waist-opening 24 into the respective leg-openings 25 and then the diaper 1 may be pulled up along the wearer's waist. Alternatively, before the front and rear waist regions 8, 9 are joined together, the respective regions of the diaper 1 may be applied on the corresponding regions of the wearer's body and then the hook elements 15, 16 may be placed into engagement with the associated loop elements 17, 18 to join the front and rear waist regions 8, 9 so as to shape the diaper 1 in the pull-on pants. Thus the diaper 1 according to the present invention is convenient to put it on the wearer's body selectively in pull-on pants fashion or open-type diaper fashion.

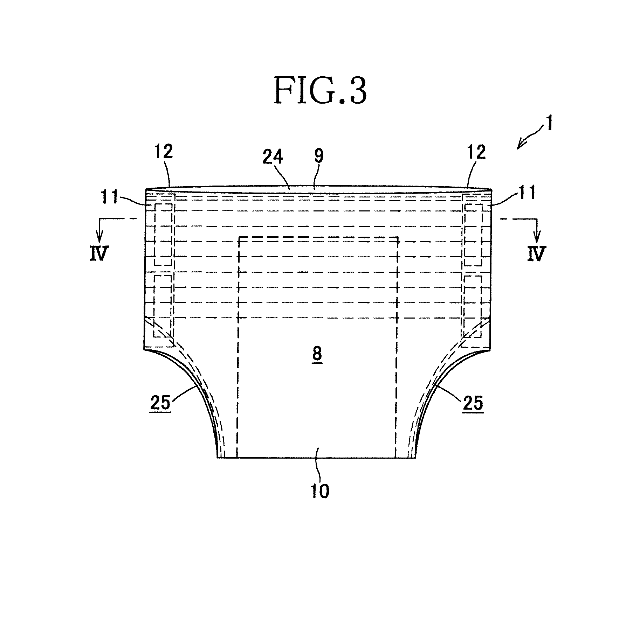

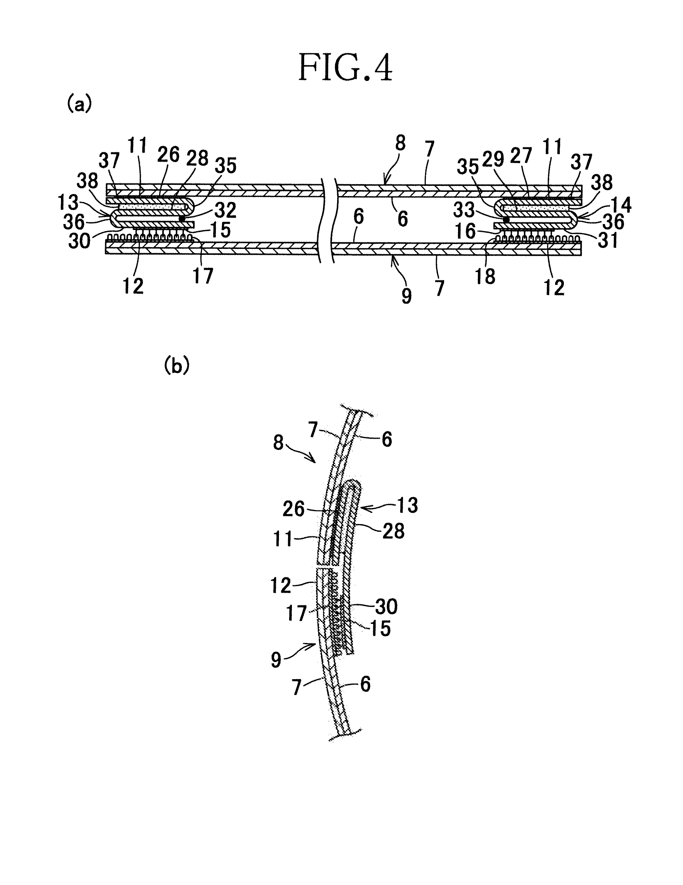

Fig. 3 is a perspective view showing the diaper before it is practically used and FIG. 4 (a) shows a partially cutaway sectional view taken along a line IV-IV in Fig. 3. Before practically used, such diaper 1 is in collapsed state, i.e., the front and rear waist regions 8, 9 are put flat together with the top-sheet 6 being in face-to-face relationship. The mount members 13, 14 are folded back from the side of the top-sheet 6 of the front waist region 6 to have a substantially Z-shaped cross-section and a substantially inverted Z-shaped cross-section, respectively, and thereby to define first regions 26, 27, second regions 28, 29 and third regions 30, 31. Specifically, the mount members 13, 14 are folded along first folding lines 35, 35 and second folding lines 36, 36 extending in the longitudinal direction Y so that each of the first regions 26, 27 extends from one transversely outer end of the mount member 13 or 14 to the associated first folding line 35, each of the second regions 28, 29 extends from the associated first folding line 35 to the associated second folding line 36 and each of the third region 30, 31 extends from the associated second folding line 36 to the other transversely outer end of the mount member 13 or 14. The first regions 26, 27 are bonded to the top-sheet 6 of the first waist region 8 by means of fixing layers 37 and to the second regions 28, 29 by means of second bonding layers 38.

Between the second regions 28, 29 and the third regions 30, 31, there are formed first arrays of adhesive spots 32, 33 serving for temporarily fixing these second regions 28, 29 to the third regions 30, 31 and thereby to prevent theses second and second regions 28, 29, 32, 33 from being unfolded in untimely moment in the course of making the diaper 1. The first arrays of adhesive spots 32, 33 are broken upon completion of the product or when the diaper 1 is put on the wearer's body and the diaper 1 presents a shape as seen in Fig. 1.

The third regions 30, 31 are provided on respective sides facing the top-sheet 6 of the rear waist region 9 with the hook elements 15, 16 attached thereto by suitable bonding means (not shown). The top-sheet 6 in the rear waist region 9 are provided with the loop elements 17, 18 attached thereto by suitable bonding means (not shown) and put in engagement with the associated hook elements 15, 16.

In the construction as has been described above, the first arrays of adhesive spots 32, 33 are formed adjacent the respective inner ends of the mechanical fastener assemblies so that these adhesive spots 32, 33 may overcome the associated hook elements 15, 16, preferably may overcome the hook elements 15, 16 engaged with the loop elements 17, 18.

FIG. 4 (b) shows only the mount member 13 having been released from the temporarily fixing effect of the first array of adhesive spots 32 since the state of the mount member 14 after released from the effect of the associated first array of adhesive spots 33 is similar to the case of the mount member 14. To release the mount member 13 from the temporarily fixed state as shown by FIG. 4 (a), the top-sheet 6 in the front waist region 8 may be drawn apart from the top-sheet 6 in the rear waist region 9 with the hands inserted between the front and rear waist regions 8, 9 or with the fingers gripping the top-and back-sheets 6, 7 of the front and rear waist regions 8, 9.

The first arrays of adhesive spots 32, 33 temporarily fix the second regions 28, 29 to the third regions 30, 31. The second regions 28, 29 are bonded to the top-sheet 6 by the intermediary of the second adhesive layers 38, 38, the first regions 26, 27 and the first adhesive layers 37, 37. The top-sheet 6 is lapped over the second regions 28, 29 by means of the respective adhesive layers and, in consequence, the second regions 28, 29 are provided with stiffness sufficient to support the first arrays of adhesive spots 32, 33 reliably on the side of the top-sheet 6 in the front waist region 8.

The third regions 30, 31 are bonded to the hook elements 15, 16 and, in consequence, the third regions 30, 31 are also provided with stiffness sufficient to support the first arrays of adhesive spots 32, 33 on the side of the top-sheet 6 in the rear waist region 9. In this way, when the second regions 28, 29 are drawn apart from the third regions 30, 31, there is no anxiety that the first arrays of adhesive spots 32, 33 might follow the second regions 28, 29 of the third regions 30, 31 and thereby make it impossible to release the mount members 32, 33 from the temporarily fixing effect of the first arrays of adhesive spots 32, 33.

In addition, by forming the first arrays of adhesive spots 32, 33 so as to lap over the regions in which the hook elements 15, 16 are engaged with the loop elements 17, 18 and, in consequence, the third regions 30, 31 are provided with correspondingly higher stiffness. As a result, such high stiffness serves to restrict the possibility that the third regions 30, 31 might follow the movement of the first arrays of adhesive spots 32, 33 as these first arrays of adhesive spots 32, 33 are pulled upward.

The second regions 28, 29 are bonded to the top-sheet 6 in the front waist region 8 by the intermediary of the first regions 26, 27 and the third regions 30, 31 are bonded to the top-sheet 6 in the rear waist region 9 by the intermediary of the hook elements 15, 16 and the loop elements 17, 18 engaged together. With such arrangement, the mount members 13, 14 can be smoothly released from the temporarily fixing effect of the first arrays of adhesive spots 32, 33 by drawing apart the top-sheet 6 in the front waist region 8 from the top-sheet 6 in the rear waist region 9. The strength of engagement between the hook elements 15, 16 with the loop elements 17, 18 may be set to be higher than the adhesive strength of the first arrays of adhesive spots 32, 33 to restrict the possibility that the hook elements 15, 16 might be disengaged from the loop elements 17, 18 before the mount members 13, 14 are released from the temporarily fixing effect of the first arrays of adhesive spots 32, 33.

After the mount members 13, 14 have been released from the temporarily fixing effect of the first arrays of adhesive spots 32, 33, the third regions 30, 31 extend outward in the transverse direction X beyond the first lateral zones 11 of the front waist region 8. The third regions 30, 31 extending outward in this manner are provided on the respective outer sides facing the wearer's clothes with the hook elements 15, 16 with which the associated loop elements 17, 18 are engaged. With such arrangement of the hook elements 15, 16 and the loop elements 17, 18, the hook elements 15, 16 are smoothly engaged with the associated loop elements 17, 18 when the diaper 1 is put on the wearer's body.

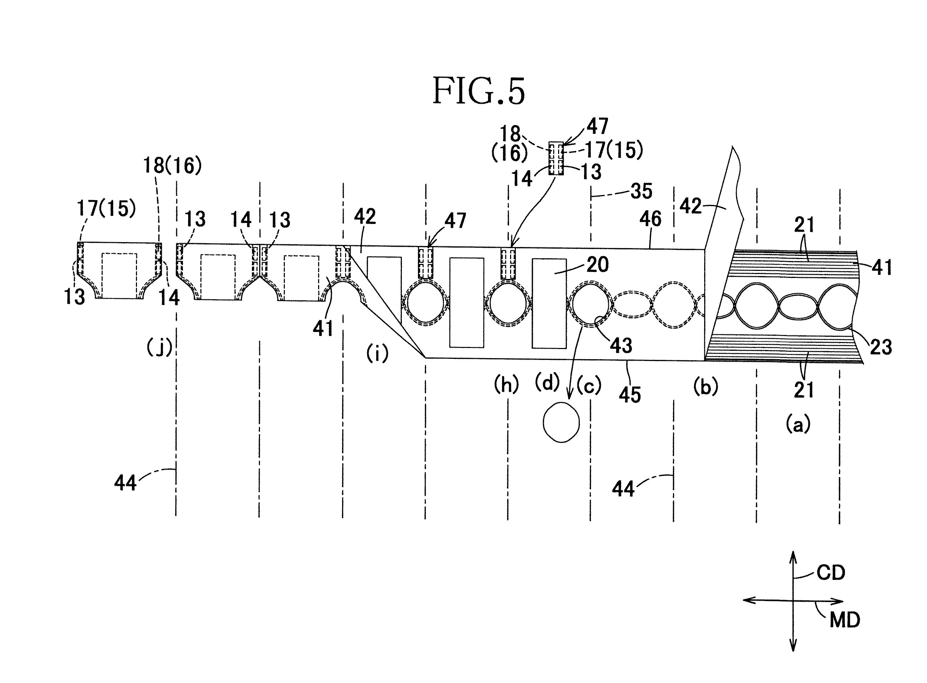

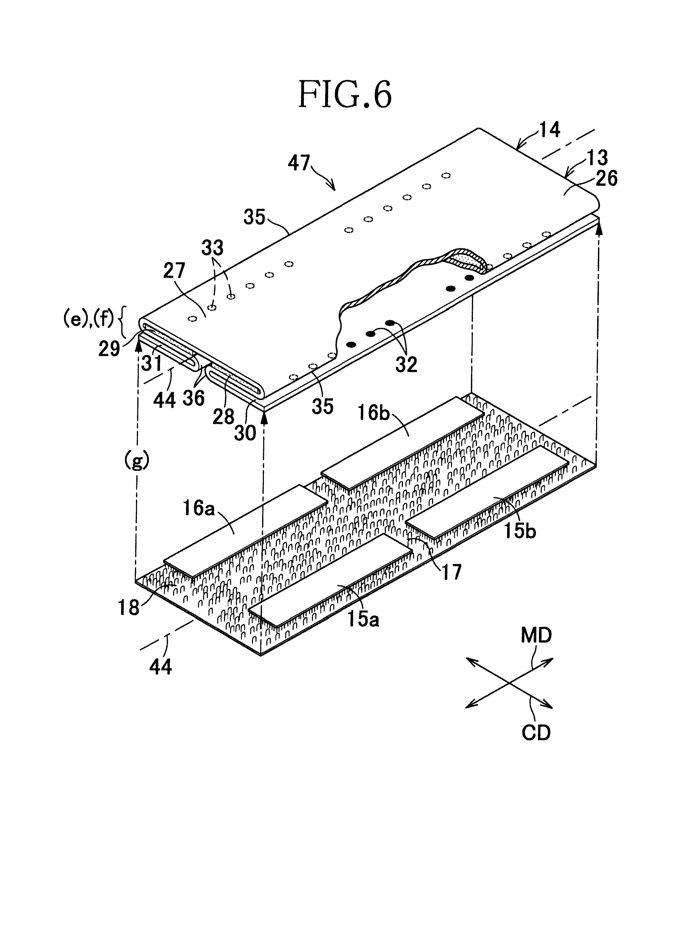

Figs. 5 and 6 schematically illustrate the method of making the diaper 1. As schematically illustrated by Fig. 5, web comprising diapers 1 is formed in machine direction MD and finally severed along a cutting line 44 extending in a cross direction CD which is orthogonal to the machine direction MD to output the individual diapers 1. In the web comprising the diapers 1, the second lateral zones 12 and the first lateral zones 11 of the diapers 1 are respectively continuous one to another so that the transverse direction X of the diapers 1 coincides with the machine direction MD.

In a first step (a), first web 41 used to form the back-sheet 7 are fed and the waist-surrounding elastic members 21 and the leg-surrounding elastic members 23 are continuously attached to this first web 41 under tension in the machine direction MD. It should be appreciated that joining means such as adhesive used to attach these elastic members 21, 23 are not illustrated in Fig. 3.

In a second step (b), second web 42 used to form the top-sheet 6 is fed toward and bonded to the first web 41. Both the first web 41 and the second web 42 are formed by fibrous webs. With respect to this second step (b) and later steps, the waist-surrounding elastic members 21 are not illustrated and the leg-surrounding members 23 are indicated by dashed lines in the accompanying diagrams.

In a third step (c), the first and second webs 41, 42 are formed with a circular opening 43 along a circle defined by the leg-surrounding elastic members 23 so that peripheral edges of these opening 43 may define the peripheral edges 22 of the leg-openings 25. The cutting line 44 extends diametrically across the opening 43.

In a fourth step (d), the liquid-absorbent core 20 is attached to the second web 42 by joining means (not shown) between each pair of the adjacent openings 43, 43 in the machines direction MD.

Fig. 6 is a diagram schematically illustrating fifth, sixth and seventh steps (e), (f), (g) of forming a fastener assembly comprising the mount members 13, 14, the hook elements 15, 16 and the loop elements 17, 18.

In the fifth step (e), the mount members 13, 14 are folded back to form first regions 26, 27, second regions 28, 29, and third regions 30, 31, respectively. The mount members 13, 14 are respectively formed by single fibrous non-woven fabric sheets and folded back along a first folding line 35 and a second folding line 36 both extending in the machine direction MD. It should be appreciated here that, in the web comprising the continuously made diapers, each pair of the adjacent mount members 13, 14 are formed at once and therefore the first regions 26, 27 are continuously formed in the cross direction CD. These first regions 26, 27 are subsequently cut off along the cutting line 44.

In the sixth step (f), the first arrays of adhesive spots 32, 33 are formed between the second regions 28, 29 and the third regions 30, 31 of the folded mount members 13, 14 for temporarily fixing the second regions 28, 29 and the third regions 30, 31. It should be appreciated that the fifth step and the sixth step may be set so as to be concurrently developed. The first arrays of adhesive spots 32, 33 are intermittently formed substantially over the entire area of the mount members 13, 14 as viewed in the machine direction MD. By intermittently forming the first arrays of adhesive spots 32, 33 in this manner, it is possible to prevent the mount members 13, 14 from having unacceptably high stiffness and consequently the wearer might experience uncomfortable feeling.

In the seventh step (g), the hook elements 15, 16 are attached to the respective third regions 30, 31 of the mount members 13, 14. The hook elements 15, 16 may be previously engaged with the associated loop elements 17, 18 or the hook elements 15, 16 may be engaged with the associated loop elements 17, 18 after the hook elements 15, 16 have been attached to the associated third regions 30, 31. The loop elements 17, 18 are continuous in the cross direction CD and subsequently cut off along the cutting line 44.

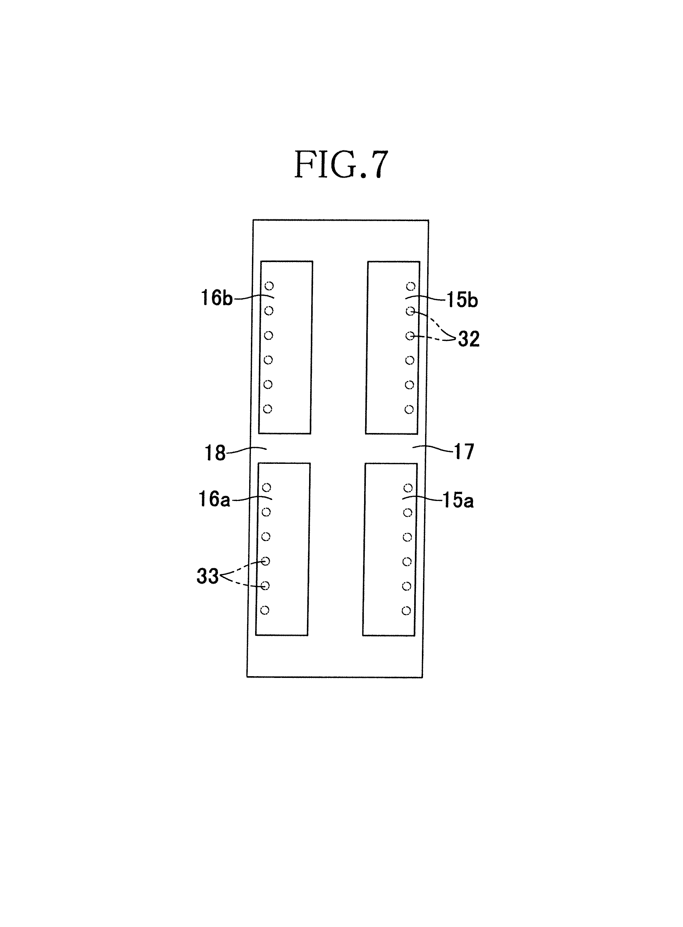

Fig. 7 illustrates a position relationship among the first arrays of adhesive spots 32, 33, the hook elements 15, 16 and the loop elements 17, 18. In Fig. 7, the mount member 14 is eliminated for convenience of illustration and the first arrays of adhesive spots 32, 33 are indicated by imaginary lines. The first arrays of adhesive spots 32, 33 are formed in the mechanical fastener assemblies in which the hook elements 15, 16 are lapped over and engaged with the associated loop elements 17, 18. In consideration of undesired irritation to the wearer's skin and smooth engagement, the hook elements 15, 16 are dimensioned to be smaller than the loop elements 17, 18. As a result, the loop elements 17, 18 extend outward beyond peripheries of the associated hook elements 15, 16 and it is desired the first arrays of adhesive spots 32, 33 are absent in these surplus regions of the loop elements 17, 18 and the presence of these first arrays of adhesive spots 32, 33 are limited to the regions in which the hook elements 15, 16 are engaged with the associated loop elements 17, 18. In other words, the surplus regions of the loop elements 17, 18 are present outside the hook elements 15, 16 as viewed in the machine direction MD but the first arrays of adhesive spots 32, 33 are not present in these surplus regions. More specifically, the surplus regions of the loop elements 17, 18 extending outward beyond the peripheries of the hook elements 15, 16 are respectively dimensioned in the machine direction MD to be approximately 10 mm. Between the hook elements 15a, 16a and the hook elements 15b, 16b, regions having no hook element are defined and, in these regions also, none of the first arrays of adhesive spots is formed. None of the first arrays of adhesive spots 13, 14 is formed in the regions having none of hook elements 15, 16, in other words, in the regions in which the hook elements 15, 16 are not engaged with the loop elements 17, 18 and thereby the mount members 13, 14 can be maintained flexible.

However, it is not essential to form the first arrays of adhesive spots 32, 33 so as to lap over the hook elements but the arrays of adhesive spots may be partially located in the regions having none of the hook elements.

The mechanical fastener assembly 47 formed in this manner is fed toward the second web 42 in the eighth step (h) and, immediately before such feeding, the assembly is turned by 90° the mechanical fastener assembly In a eighth step (h), the fastener assembly 47 constructed as has been described above is fed toward the second web 42 as illustrated by Fig. 3. It should be understood that the fastener assembly 47 is turned by 90°. In consequence, the machine direction MD as well as the cross direction CD indicated in Fig. 6 is reversed in Fig. 5. The first and second webs 41, 42 define one end 45 and the opposite end 46 both extending in the machine direction MD so that a section of the first and second webs 41, 42 extending aside toward the end 45 will define the front waist region 8 and a section thereof extending aside toward the opposite end 46 will define the rear waist region 9 of the diaper 1. The mount members 13, 14 are bonded to the portion of the second web 42 extending aside toward the opposite end 46 by means of adhesive layer (not shown).

In a ninth step (i), the web assembly comprising the first and second webs 41, 42 is folded in two with the opposite ends 45, 46 exactly placed upon each other and with the liquid-absorbent core 20 inside. The loop elements 17, 18 are bonded to the second web 42 at regions thereof placed aside toward the end 45 in the course of folding the web assembly comprising the first and second webs 41, 42 into two.

In a tenth step (j), the web assembly comprising the first and second webs 41, 42 folded in two is cut along the cutting line 44 to obtain the individual diaper 1 wherein the first lateral zones 11 of the front waist region 8 are defined by the end 45 of the web assembly comprising the first and second webs 41, 42 and the second lateral zones 12 of the rear waist region 9 are defined by the opposite end 46 of the web assembly. Thereupon, the mount members 13, 14 and the loop elements 17, 18 are also cut along the cutting line 44, respectively, so that the mount members 13, 14 extend along the first lateral zones 11 and the loop elements 17, 18 extend along the second lateral zones 12. The machine direction MD and the cross direction CD in the process of making the diaper 1 correspond to the transverse direction X and the longitudinal direction Y of the diaper 1, respectively.

According to the method as has been described above, the second regions 28, 29 are temporarily fixed to the third regions 30, 31 by the first arrays of adhesive spots 32, 33 to prevent the undesired situation in which the second regions 28, 29 and the third regions 30, 31 might be unfolded in untimely moment. The third regions 30, 31 unstuck from the second regions 28, 29 would flap in the course of feeding and would be sometimes caught by feeding rollers or the like. The mount members 13, 14 caught by the rollers may cause a failure of the equipment or a displacement of the web assembly. Such possibility or apprehension can be avoided by forming the first arrays of adhesive spots 32, 33 according to the present invention. To prevent the third regions 32, 33 from flapping, it is desired to form the first arrays of adhesive spots 32, 33 in regions as remote as possible from the second folding line 36. Specifically, the first arrays of adhesive spots 23, 33 are preferably formed on the respective inner ends of the hook elements 15, 16 as viewed in the transverse direction X.

The first arrays of adhesive spots 32, 33 may be formed by adhesive such as hot melt adhesive, thermal- or ultrasonic bond. In the case of the illustrated embodiment, each pair of the adjacent adhesive spots 32, 33 is spaced from each other by about 10 mm in the longitudinal direction Y. With such arrangement, an adhesive strength between the second regions 28, 29 and the associated third regions 30, 31 of the mount members 13, 14 are preferably in a range of 0.2 to 3.0 N/80 mm. The adhesive strength was measured by the method as will be described below.

A plurality of diapers 1 each having the first arrays of adhesive spots 32, 33, and the hook elements 15, 16 engaged with the associated loop elements 17, 18 as shown in Figs. 3 and 4 are prepared. Each of the diapers 1 was cut off between the hook elements 15a and 15b or 16a and 16b in the transverse direction X and then the folded third regions 30, 31 are cut off in the longitudinal direction Y along the respective inner sides as viewed in the transverse direction Y to obtain test pieces. These samples were successively subjected to the measurement. Specifically, the test piece was held by chucks of the tensile tester (Autograph of AG-X series) manufactured by SHIMADZU CORPORTION) at positions 50 mm from the end on the waist-opening side and the maximum peel strength was determined with a inter-chuck distance of 50 mm and a peel rate of 500 mm/min. From a plurality of measurements, an average value was obtained as the adhesive strength.

The adhesive strength as has been described above is set to ensure that the function of the first arrays of adhesive spots 32, 33 is effective in the course of making the diaper 1 to maintain the second regions 28, 29 and the third regions 30, 31 in temporarily fixed condition and to be lower than the engagement strength between the hook elements 15, 16 and the loop elements 17, 18. In this way, the temporary fixing can be kept in the course of making the diaper 1 but only the temporary fixing effect can be removed without disengaging the hook elements 15, 16 with the associated loop elements 17, 18 when the diaper 1 is put on the wearer's body.

While the hook elements 15, 16 are attached to the associated mount members 13, 14 and then the loop elements 17, 18 are attached to the hook elements 15, 16 to form the fastener assembly which is then attached to the second web 42 constituting the chassis 2 according to the embodiment as has been described, the invention is not limited to this embodiment. Alternatively, the hook elements 15, 16 may be attached to the second web 42 on its region put aside toward the end 46 thereof and the loop elements 17, 18 may be attached to the second web 42 on its region put aside toward the end 45 and thereafter the web assembly may be folded in two so as to engage the hook elements 15, 16 with the associated loop elements 17, 18.

For the method of making the diaper 1 as has been described above, various timings for feeding of the liquid-absorbent core 20, forming of the opening 37 which define the peripheral edges of the respective leg-openings 25 and the other steps may be appropriately changed.

While the hook elements 15, 16 are attached to the front waist region 8 and the loop elements 17, 18 are attached to the rear waist region 9 according to the embodiment as has been described above, it is possible to reverse this relationship. Specifically, while the front waist region 8 has been designated as the first waist region and the rear waist region 9 has been designated as the second waist region, it is possible to designate the front waist region 8 as the second waist region and to designate the rear waist region 9 as the first waist region. In any case, it is desired to attach the hook elements to the second web 42 in a manner free from the anxiety that the hook elements having relatively high stiffness might come in contact with the wearer's skin and cause any damage for the wearer's skin.

While the diaper 1 includes the liquid-absorbent core 20 prepared separately of the chassis 2 according to the embodiment as has been described above, it is possible to sandwich the liquid-absorbent core between the top- and back-sheets 6, 7 of the chassis 2.

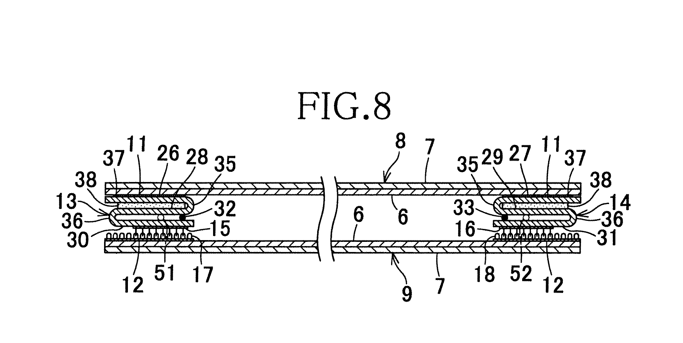

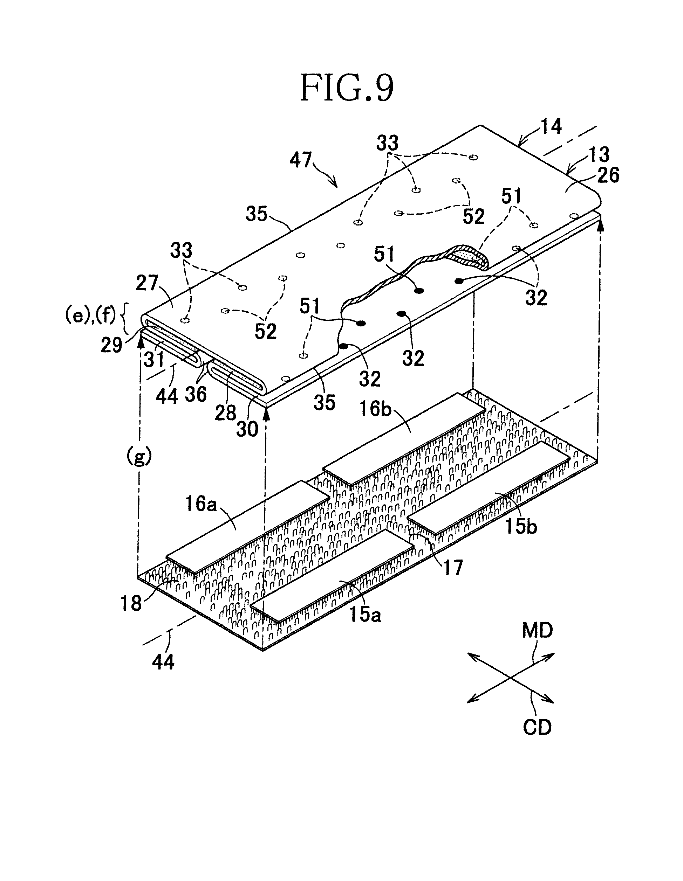

Figs. 8 and 9 illustrate an alternative embodiment similar to the previously described embodiment except that this embodiment comprises, in addition to the first arrays of adhesive spots 32, 33, second arrays of adhesive spots 51, 52. The features other than these second arrays of adhesive spots 51, 52 are similar to those in the previously described embodiment and detailed description thereof will be eliminated. In the alternative embodiment, the members as well as the elements similar to those in the previous embodiment are designated by the similar reference numerals.

Fig. 8 is a view similar to FIG. 4 (a), showing the diaper 1 in a sectional view. As shown, the first arrays of adhesive spots 32, 33 and the second arrays of adhesive spots 51, 52 are formed between the second regions 28, 29 and the third regions 30, 31 of the respective mount members 13, 14. The second arrays of adhesive spots 51, 52 are placed aside outward from the first arrays of adhesive spots 32, 33 as viewed in the transverse direction X.

Fig. 9 is a view similar to Fig. 6, showing a position relationship among the mount members 13, 14, the hook elements 15, 16 and the loop elements 17, 18 in the course of making the diaper 1. The second arrays of adhesive spots 51, 52 are formed on the inward sides of the associated first arrays of adhesive spots 32, 33 as viewed in the cross direction CD. The first arrays of adhesive spots 32, 33 as well as the second arrays of adhesive spots 51, 52 are intermittently arranged in the machine direction MD in a manner that the first arrays of adhesive spots 32, 33 as well as the second arrays of adhesive spots 51, 52 are respectively spaced at the intervals of approximately 30 mm. The first arrays of adhesive spot 32, 33 are spaced from the associated second arrays of adhesive spots 51, 52 by approximately 6 mm in the cross direction CD.

Though not lining up, the individual adhesive spots in the second arrays of adhesive spots 51, 52 are located between respective pairs of the adhesive spots adjacent in the machine direction MD in the first arrays of adhesive spots 32, 33 so that the individual adhesive spots the first arrays 32, 33 and the second arrays 51, 52 never lap over one on another in the cross direction CD.

The machine direction MD and the cross direction CD of these first arrays of adhesive spots 32, 33 as well as the second arrays of adhesive spots 51, 52 respectively correspond to the longitudinal direction Y and the transverse direction X, respectively, in the diaper 1 as the product.

With the arrangement as has been described just above, the first arrays of adhesive spots 32, 33 cooperate with the second arrays of adhesive spots 51, 52 for temporary fixing. In advantageous consequence, the temporary fixing strength of the respective arrays of adhesive spots 32, 33, 51, 52 may be reduced without an anxiety that the mount members might be unintentionally released from the temporarily fixing effect of these arrays of adhesive spots. Relatively low temporary fixing strength facilitates the mount members to be released from the temporarily fixing effect without being accompanied with disengagement between the hook elements 15, 16 and the loop elements 17, 18.

By additionally forming the second arrays of adhesive spots 51, 52, the adjacent spots in the respective arrays of adhesive spots 32, 33, 51, 52 can be further spaced from each other and the number of these spots can be correspondingly decreased. Consequently, undesired increase of the stiffness due to formation of the arrays of adhesive spots can be restricted.

The adhesive strength in this embodiment including the first arrays of adhesive spots 32, 33 and the second arrays of adhesive spots 51, 52 is set in the same range of 0.2 to 3.0 N/80 mm as in the case of the previously described embodiment. The method of measuring the adhesive strength also is similar to that as has been previously described.

With the arrangement of the first arrays of adhesive spots 32, 33 and the second arrays of adhesive spots 51, 52 such that the individual adhesive spots of the first and second arrays never lap over on each other in the cross direction CD, the individual adhesive spots of the first arrays 32, 33 and the second arrays 51, 52 are alternately broken in the course of releasing the mount members from the temporarily fixing effect of these arrays of adhesive spots. In this way, the mount members can be easily released from the effect of these arrays of adhesive spots.

While the first arrays of adhesive spots 32, 33 are described to be spaced in the machine direction MD from the second arrays of adhesive spots 51, 52 by approximately 30 mm, more generally, it is desired to space the first arrays 32, 33 from the associated second arrays 51, 52 by 20 mm or longer. This is for the reason that, if this distance is less than 20 mm, it will be difficult to break the first arrays and the associated second arrays alternately. Specifically, the individual adhesive spots in the first arrays and the associated second arrays must be sometimes broken at once with correspondingly increased effort.

The second arrays of adhesive spots 51, 52 may be formed at the same time and in the same manner at and in which the first arrays of adhesive spots 31, 32 are formed.

## Claims

1. A method of making a wearing article comprising a chassis (2) and mechanical fasteners (3) as basic components, said chassis (2) having a longitudinal direction (Y), a transverse direction (X), a side (4) facing wearer's skin and a side (5) facing wearer's clothes, a first waist region (8) corresponding to one of front and rear waist regions, a second waist region (9) corresponding to the other of said front and rear waist regions and a crotch region (10) extending between said first and second waist regions (8, 9), and said mechanical fasteners (3) include first mechanical fastener members (15, 16) provided along a pair of first lateral zones (11) of said first waist region (8) opposed to each other in said transverse direction (X) so as to extend in the longitudinal direction (Y) and second mechanical fastener members (17,18) provided along a pair of second lateral zones (12) of said second waist region (9) opposed to each other in said transverse direction (X) so as to extend in said longitudinal direction (Y) wherein said first mechanical fastener members (15,16) are detachably engaged with said second mechanical fastener members (17,18) and wherein said first mechanical fastener members (15,16) are formed along said first lateral zones (11) by the intermediary of mount members (13,14), said method comprising the steps of:
folding back each of said mount members (13,14) along a first folding line (35) extending in said longitudinal direction (Y) to define a first region (26,27) and a second region (28,29), then along a second folding line (36) also extending in said longitudinal direction (Y) to define said second region (28,29) and a third region (30,31) so that said second region (28,29) may be lapped on said first region (26,27) while said third region (30,31) may be lapped on said second region (28,29);
forming an array of adhesive spots (32,33) between said second region (28,29) and said third region (30,31) for temporarily fixing said second region (28,29) to said third region (30,31);
bonding said third region (30,31) to associated said first mechanical fastener member (15,16);
engaging said first mechanical fastener member (15,16) with associated said second mechanical fastener member (17,18);
bonding said first region (26,27) to associated said first lateral zones (11); and
bonding said second mechanical fastener member (17,18) to associated said second lateral zone (12);
**characterized in that** the first mechanical fastener members are respectively divided into upper halves (15a, 16a) and lower halves (15b, 16b) in the longitudinal direction (Y) so as to form spaces therebetween; the arrays of adhesive spots (32, 33) are formed substantially over a full length of said mount member (13, 14) in said longitudinal direction (Y); and the arrays of adhesive spots (32,33) are formed in regions overlapping with the upper and lower halves (15a, 15b; 16a, 16b) of the first mechanical fastener members and in regions overlapping with said spaces.

2. A method of making a wearing article comprising a chassis (2) and mechanical fasteners (3) as basic components, said chassis (2) having a longitudinal direction (Y), a transverse direction (X), a side (4) facing wearer's skin and a side (5) facing wearer's clothes, a first waist region (8) corresponding to one of front and rear waist regions, a second waist region (9) corresponding to the other of said front and rear waist regions and a crotch region (10) extending between said first and second waist regions (8,9), and said mechanical fasteners (3) include first mechanical fastener members (15,16) provided along a pair of first lateral zones (11) of said waist region (8) opposed to each other in said transverse direction (X) so as to extend in said longitudinal direction (Y) and second mechanical fastener members (17,18) provided along a pair of second lateral zones (12) of said second waist region (9) opposed to each other in said transverse direction (X) so as to extend in said longitudinal direction (Y) wherein said first mechanical fastener members (15,16) are detachably engaged with said second mechanical fastener members (17,18) and wherein said first mechanical fastener members (15,16) are formed along said first lateral zones (11) by the intermediary of mount members (13,14), said method comprising the steps of:
folding back each of said mount members (13,14) along a first folding line (35) extending in said longitudinal direction (Y) to define a first region (26,27) and a second region (28,29), then along a second folding line (36) also extending in said longitudinal direction (Y) to define said second region (28,29) and a third region (30,31) so that said second region (28,29) may be lapped on said first region (26,27) while said third region (30,31) may be lapped on said second region (28,29);
forming an array of adhesive spots (32,33) between said second region (28,29) and said third region (30,31) for temporarily fixing said second region (28,29) to said third region (30,31);
engaging said first mechanical fastener member (15,16) with associated said second mechanical fastener member (17,18);
bonding said third region (30,31) to associated said first mechanical fastener member (15,16) engaged with said second mechanical fastener member (17,18);
bonding said first region (26,27) to associated said first lateral zone (11); and
bonding said second mechanical fastener member (17,18) to associated said second lateral zone (12);
**characterized in that** the first mechanical fastener members are respectively divided into upper halves (15a, 16a) and lower halves (15b, 16b) in the longitudinal direction (Y) so as to form spaces therebetween; the arrays of adhesive spots (32, 33) are formed substantially over a full length of said mount member (13, 14) in said longitudinal direction (Y); and the arrays of adhesive spots (32,33) are formed in regions overlapping with the upper and lower halves (15a, 15b; 16a, 16b) of the first mechanical fastener members and in regions overlapping with said spaces.

3. The method of making the wearing article according to Claim 1 or 2, wherein a process of making said wearing article has a machine direction (MD) and a cross direction (CD) orthogonal to said machine direction (MD) and the chassis (2) of said wearing article is continuously formed so that said transverse direction (X) of said chassis (2) corresponds to said machine direction (MD), said method further includes steps of:
feeding web (41,42) forming said chassis (2) in said machine direction (MD); and
cutting said chassis (2) in said cross direction (CD) after said first mechanical fastener member (15,16) has been engaged with associated said second mechanical fastener member (17,18);
wherein said first lateral zones (11) are formed along one end of said web (41,42) extending in the machine direction (MD) and said second lateral zones (12) are formed along the opposite end.

4. The method of making the wearing article according to Claim 3, wherein respective pairs of adjacent said mount members (13,14) of said wearing articles continuously formed in said machine direction (MD) are contiguous to each other in respective said third regions (30,31) and cut off in these contiguous third regions (30,31) in said cross direction (CD).

5. A wearing article comprising a chassis (2) and mechanical fasteners (3) as basic components, said chassis (2) having a longitudinal direction (Y), a transverse direction (X), a side (4) facing wearer's skin and a side (5) facing wearer's clothes, a first waist region (8) corresponding to one of front and rear waist regions, a second waist region (9) corresponding to the other of said front and rear waist regions and a crotch region (10) extending between said first and second waist regions (8,9), and said mechanical fasteners (3) include first mechanical fastener members (15,16) provided along a pair of first lateral zones (11) of said first waist region (8) opposed to each other in said transverse direction (X) so as to extend in the longitudinal direction (Y) and second mechanical fastener members (17,18) provided along a pair of second lateral zones (12) of said second waist region (9) opposed to each other in said transverse direction (X) so as to extend in said longitudinal direction (Y) wherein said first mechanical fastener members (15,16) are detachably engaged with said second mechanical fastener members (17,18) and wherein said first mechanical fastener members (15,16) are formed along said first lateral zones (11) by the intermediary of mount members (13,14), said wearing article further comprising:
each of said mount members (13,14) comprises a first folding line (35), a second folding line (36), a first region (26,27) and a second region (28,29) defined by said first folding line (35) and said second folding line (36), and a third region (30,31) defined together with said second region (28,29) by said second folding line wherein said second region (28,29) is lapped on said first region (26,27) and said third region (30,31) is lapped on said second region (28,29);
said third region (30,31) is bonded to associated said first mechanical fastener member (15,16);
said first region (26,27) is bonded to associated said first lateral zone (11); and
an array of adhesive spots (32,33) is formed between said second region (28,29) and said third region (30,31) for temporarily fixing said second region (28,29) to said third region (30,31);
**characterized in that** the first mechanical fastener members are respectively divided into upper halves (15a, 16a) and lower halves (15b, 16b) in the longitudinal direction (Y) so as to form spaces therebetween; the arrays of adhesive spots (32, 33) are formed substantially over a full length of said mount member (13, 14) in said longitudinal direction (Y); and the arrays of adhesive spots (32,33) are formed in regions overlapping with the upper and lower halves (15a, 15b; 16a, 16b) of the first mechanical fastener members and in regions overlapping with said spaces.

6. The wearing article according to Claim 5, wherein said array of adhesive spots (32,33) is formed so as to lap on a region in which said first mechanical fastener member (15,16) is engaged with said second mechanical fastener member (17,18).

7. The wearing article according to Claim 5 or 6, wherein said array of adhesive spots (32,33) is formed at least along said transversely inner end of said region in which said first mechanical fastener member (15,16) is engaged with associated said second mechanical fastener member (17,18)

8. The wearing article according to any one of Claims 5 through 7, wherein said array of adhesive spots (32,33) is intermittently formed substantially over a full length of said mount member (13,14) in said longitudinal direction (Y).

9. The wearing article according to any one of Claims 5 through 8, wherein a plurality of said arrays of adhesive spots (32,33) are formed so as to be spaced one from another in said transverse direction (X).

10. The wearing article according to Claim 9, wherein said plurality of arrays of adhesive spots (27,33) are arranged so that respective pairs of the individual adhesive spots in said plurality of arrays being adjacent in said transverse direction (X) never lap on each other.

## Patentansprüche

1. Verfahren zum Herstellen eines Kleidungsstücks, umfassend ein Chassis (2) und mechanische Befestigungen (3) als Grundkomponenten, wobei das Chassis (2) eine Längsrichtung (Y), eine Querrichtung (X), eine Seite (4), die zu Haut eines Trägers zeigt, und eine Seite (5), die zu Kleidung des Trägers zeigt, einen ersten Hüftbereich (8), der entweder einem vorderen oder einem hinteren Hüftbereich entspricht, einen zweiten Hüftbereich (9), der dem jeweils andern aus dem vorderen und dem hinteren Hüftbereich entspricht, und einen Schrittbereich (10), der sich zwischen dem ersten und dem zweiten Hüftbereich (8, 9) erstreckt, aufweist, und die mechanischen Befestigungen (3) erste mechanische Befestigungselemente (15, 16) beinhalten, die entlang einem Paar erster seitlicher Zonen (11) des ersten Hüftbereichs (8) sich in der Querrichtung (X) gegenüberliegend vorgesehen sind, so dass sie sich in der Längsrichtung (Y) erstrecken, und zweite mechanische Befestigungselemente (17, 18), die entlang einem Paar zweiter seitlicher Zonen (12) des zweiten Hüftbereichs (9) sich in der Querrichtung (X) gegenüberliegend vorgesehen sind, so dass sie sich in der Längsrichtung (Y) erstrecken, wobei die ersten mechanischen Befestigungselemente (15, 16) mit den zweiten mechanischen Befestigungselementen (17, 18) in lösbaren Eingriff gebracht werden und wobei die ersten mechanischen Befestigungselemente (15, 16) entlang den ersten seitlichen Zonen (11) durch die Zwischenschaltung von Anbringungselementen (13, 14) ausgebildet sind, wobei das Verfahren die folgenden Schritte aufweist:
Zurückschlagen der jeweiligen Anbringungselemente (13, 14) entlang einer ersten Falzlinie (35), die sich in der Längsrichtung (Y) erstreckt, um einen ersten Bereich (26, 27) und einen zweiten Bereich (28, 29) zu definieren, dann entlang einer zweiten Falzlinie (36), die sich ebenfalls in der Längsrichtung (Y) erstreckt, um den zweiten Bereich (28, 29) und einen dritten Bereich (30, 31) zu definieren, so dass der zweite Bereich (28, 29) über den ersten Bereich (26, 27) gelegt werden kann, während der dritte Bereich (30, 31) über den zweiten Bereich (28, 29) gelegt werden kann;
Ausbilden einer Anordnung von Klebepunkten (32, 33) zwischen dem zweiten Bereich (28, 29) und dem dritten Bereich (30, 31) zum vorübergehenden Fixieren des zweiten Bereichs (28, 29) an dem dritten Bereich (30, 31);
Verbinden des dritten Bereichs (30, 31) mit dem zugeordneten ersten mechanischen Befestigungselement (15, 16);
In-Eingriff-Bringen des ersten mechanischen Befestigungselements (15, 16) mit dem zugeordneten zweiten mechanischen Befestigungselement (17, 18);
Verbinden des ersten Bereichs (26, 27) mit den zugeordneten ersten seitlichen Zonen (11); und
Verbinden des zweiten mechanischen Befestigungselements (17, 18) mit der zugeordneten zweiten seitlichen Zone (12);
**dadurch gekennzeichnet, dass** die ersten mechanischen Befestigungselemente in der Längsrichtung (Y) jeweils in obere Hälften (15a, 16a) und untere Hälften (15b, 16b) geteilt sind, so dass sie Zwischenräume zwischen sich ausbilden; die Anordnungen von Klebepunkten (32, 33) in der Längsrichtung (Y) im Wesentlichen über eine volle Länge des Anbringungselements (13, 14) ausgebildet sind; und die Anordnungen von Klebepunkten (32, 33) in Bereichen, die sich mit den oberen und unteren Hälften (15a, 15b; 16a, 16b) der ersten mechanischen Befestigungselemente überlappen, und in Bereichen, die sich mit den Zwischenräumen überlappen, ausgebildet sind.

2. Verfahren zum Herstellen eines Kleidungsstücks, umfassend ein Chassis (2) und mechanische Befestigungen (3) als Grundkomponenten, wobei das Chassis (2) eine Längsrichtung (Y), eine Querrichtung (X), eine Seite (4), die zu Haut eines Trägers zeigt, und eine Seite (5), die zu Kleidung des Trägers zeigt, einen ersten Hüftbereich (8), der entweder einem vorderen oder einem hinteren Hüftbereich entspricht, einen zweiten Hüftbereich (9), der dem jeweils andern aus dem vorderen und dem hinteren Hüftbereich entspricht, und einen Schrittbereich (10), der sich zwischen dem ersten und dem zweiten Hüftbereich (8, 9) erstreckt, aufweist, und die mechanischen Befestigungen (3) erste mechanische Befestigungselemente (15, 16) beinhalten, die entlang einem Paar erster seitlicher Zonen (11) des ersten Hüftbereichs (8) sich in der Querrichtung (X) gegenüberliegend vorgesehen sind, so dass sie sich in der Längsrichtung (Y) erstrecken, und zweite mechanische Befestigungselemente (17, 18), die entlang einem Paar zweiter seitlicher Zonen (12) des zweiten Hüftbereichs (9) sich in der Querrichtung (X) gegenüberliegend vorgesehen sind, so dass sie sich in der Längsrichtung (Y) erstrecken, wobei die ersten mechanischen Befestigungselemente (15, 16) mit den zweiten mechanischen Befestigungselementen (17, 18) in lösbaren Eingriff gebracht werden und wobei die ersten mechanischen Befestigungselemente (15, 16) entlang den ersten seitlichen Zonen (11) durch die Zwischenschaltung von Anbringungselementen (13, 14) ausgebildet sind, wobei das Verfahren die folgenden Schritte aufweist:
Zurückschlagen der jeweiligen Anbringungselemente (13, 14) entlang einer ersten Falzlinie (35), die sich in der Längsrichtung (Y) erstreckt, um einen ersten Bereich (26, 27) und einen zweiten Bereich (28, 29) zu definieren, dann entlang einer zweiten Falzlinie (36), die sich ebenfalls in der Längsrichtung (Y) erstreckt, um den zweiten Bereich (28, 29) und einen dritten Bereich (30, 31) zu definieren, so dass der zweite Bereich (28, 29) auf den ersten Bereich (26, 27) gelegt werden kann, während der dritte Bereich (30, 31) auf den zweiten Bereich (28, 29) gelegt werden kann;
Ausbilden einer Anordnung von Klebepunkten (32, 33) zwischen dem zweiten Bereich (28, 29) und dem dritten Bereich (30, 31) zum vorübergehenden Fixieren des zweiten Bereichs (28, 29) an dem dritten Bereich (30, 31);
In-Eingriff-Bringen des ersten mechanischen Befestigungselements (15, 16) mit dem zugeordneten zweiten mechanischen Befestigungselement (17, 18);
Verbinden des dritten Bereichs (30, 31) mit dem zugeordneten ersten mechanischen Befestigungselement (15, 16), das mit dem zweiten mechanischen Befestigungselement (17, 18) in Eingriff ist;
Verbinden des ersten Bereichs (26, 27) mit der zugeordneten ersten seitlichen Zone (11);
Verbinden des zweiten mechanischen Befestigungselements (17, 18) mit der zugeordneten zweiten seitlichen Zone (12);
**dadurch gekennzeichnet, dass** die ersten mechanischen Befestigungselemente in der Längsrichtung (Y) jeweils in obere Hälften (15a, 16a) und untere Hälften (15b, 16b) geteilt sind, so dass sie Zwischenräume zwischen sich ausbilden; die Anordnungen von Klebepunkten (32, 33) in der Längsrichtung (Y) im Wesentlichen über eine volle Länge des Anbringungselements (13, 14) ausgebildet sind; und die Anordnungen von Klebepunkten (32, 33) in Bereichen, die sich mit den oberen und unteren Hälften (15a, 15b; 16a, 16b) der ersten mechanischen Befestigungselemente überlappen, und in Bereichen, die sich mit den Zwischenräumen überlappen, ausgebildet sind.

3. Verfahren zum Herstellen des Kleidungsstücks gemäß Anspruch 1 oder 2, wobei ein Prozess zum Herstellen des Kleidungsstücks eine Maschinenrichtung (MD) und ein Querrichtung (CD) senkrecht zur Maschinenrichtung (MD) hat und das Chassis (2) des Kleidungsstücks kontinuierlich ausgebildet wird, so dass die Querrichtung (X) des Chassis (2) der Maschinenrichtung (MD) entspricht, wobei das Verfahren ferner die folgenden Schritte aufweist:
Transportieren eines Vlieses (41, 42), das das Chassis (2) bildet, in der Maschinenrichtung (MD); und
Schneiden des Chassis (2) in der Querrichtung (CD), nachdem das erste mechanische Befestigungselement (15, 16) mit dem zugeordneten zweiten mechanischen Befestigungselement (17, 18) in Eingriff gebracht wurde;
wobei die ersten seitlichen Zonen (11) entlang einem Ende des Vlieses (41, 42), sich in der Maschinenrichtung (MD) erstreckend ausgebildet werden und die zweiten seitlichen Zonen (12) entlang dem gegenüberliegenden Ende ausgebildet werden.

4. Verfahren zum Herstellen des Kleidungsstücks gemäß Anspruch 3, wobei jeweilige Paare benachbarter Anbringungselemente (13, 14) der Kleidungsstücke, die kontinuierlich in der Maschinenrichtung (MD) ausgebildet werden, in den jeweiligen dritten Bereichen (30, 31) durchgehend sind und in diesen durchgehenden dritten Bereichen (30, 31) in der Querrichtung (CD) abgeschnitten werden.

5. Kleidungsstück, umfassend ein Chassis (2) und mechanische Befestigungen (3) als Grundkomponenten, wobei das Chassis (2) eine Längsrichtung (Y), eine Querrichtung (X), eine Seite (4), die zu Haut eines Trägers zeigt, und eine Seite (5), die zu Kleidung des Trägers zeigt, einen ersten Hüftbereich (8), der entweder einem vorderen oder einem hinteren Hüftbereich entspricht, einen zweiten Hüftbereich (9), der dem jeweils andern aus dem vorderen und dem hinteren Hüftbereich entspricht, und einen Schrittbereich (10), der sich zwischen dem ersten und dem zweiten Hüftbereich (8, 9) erstreckt, aufweist, und die mechanischen Befestigungen (3) erste mechanische Befestigungselemente (15, 16) beinhalten, die entlang einem Paar erster seitlicher Zonen (11) des ersten Hüftbereichs (8) sich in der Querrichtung (X) gegenüberliegend vorgesehen sind, so dass sie sich in der Längsrichtung (Y) erstrecken, und zweite mechanische Befestigungselemente (17, 18), die entlang einem Paar zweiter seitlicher Zonen (12) des zweiten Hüftbereichs (9) sich in der Querrichtung (X) gegenüberliegend vorgesehen sind, so dass sie sich in der Längsrichtung (Y) erstrecken, wobei die ersten mechanischen Befestigungselemente (15, 16) mit den zweiten mechanischen Befestigungselementen (17, 18) in lösbaren Eingriff gebracht werden und wobei die ersten mechanischen Befestigungselemente (15, 16) entlang den ersten seitlichen Zonen (11) durch die Zwischenschaltung von Anbringungselementen (13, 14) ausgebildet sind, wobei das Kleidungsstück ferner umfasst:
jedes der Anbringungselemente (13, 14) umfasst eine erste Falzlinie (35), eine zweite Falzlinie (36), einen ersten Bereich (26, 27) und einen zweiten Bereich (28, 29), die von der ersten Falzlinie (35) und der zweiten Falzlinie (36) definiert werden, und einen dritten Bereich (30, 31), der zusammen mit dem zweiten Bereich (28, 29) durch die zweite Falzlinie definiert wird, wobei der zweite Bereich (28, 29) auf den ersten Bereich (26, 27) gelegt wird und der dritte Bereich (30, 31) auf den zweiten Bereich (28, 29) gelegt wird;
der dritte Bereich (30, 31) mit dem zugeordneten ersten mechanischen Befestigungselement (15, 16) verbunden ist;
der erste Bereich (26, 27) mit der zugeordneten ersten seitlichen Zone (11) verbunden ist; und
eine Anordnung von Klebepunkten (32, 33) zwischen dem zweiten Bereich (28, 29) und dem dritten Bereich (30, 31) ausgebildet ist, um den zweiten Bereich (28, 29) vorübergehend an dem dritten Bereich (30, 31) zu fixieren;
**dadurch gekennzeichnet, dass** die ersten mechanischen Befestigungselemente in der Längsrichtung (Y) jeweils in obere Hälften (15a, 16a) und untere Hälften (15b, 16b) geteilt sind, so dass sie Zwischenräume zwischen sich ausbilden; die Anordnungen von Klebepunkten (32, 33) in der Längsrichtung (Y) im Wesentlichen über eine volle Länge des Anbringungselements (13, 14) ausgebildet sind; und die Anordnungen von Klebepunkten (32, 33) in Bereichen, die sich mit den oberen und unteren Hälften (15a, 15b; 16a, 16b) der ersten mechanischen Befestigungselemente überlappen, und in Bereichen, die sich mit den Zwischenräumen überlappen, ausgebildet sind.

6. Kleidungsstück gemäß Anspruch 5, wobei die Anordnung von Klebepunkten (32, 33) so ausgebildet ist, dass sie sich auf einen Bereich legt, in dem das erste mechanische Befestigungselement (15, 16) mit dem zweiten mechanischen Befestigungselement (17, 18) in Eingriff ist.

7. Kleidungsstück gemäß Anspruch 5 oder 6, wobei die Anordnung von Klebepunkten (32, 33) mindestens entlang dem in Querrichtung inneren Ende des Bereichs ausgebildet ist, in dem das erste mechanische Befestigungselement (15, 16) mit dem zugeordneten zweiten mechanischen Befestigungselement (17, 18) in Eingriff ist.

8. Kleidungsstück gemäß einem der Ansprüche 5 bis 7, wobei die Anordnung von Klebepunkten (32, 33) diskontinuierlich im Wesentlichen über eine volle Länge des Anbringungselements (13, 14) in der Längsrichtung (Y) ausgebildet ist.

9. Kleidungsstück gemäß einem der Ansprüche 5 bis 8, wobei mehrere Anordnungen von Klebepunkten (32, 33) so angeordnet sind, dass sie in der Querrichtung (X) voneinander beabstandet sind.

10. Kleidungsstück gemäß Anspruch 9, wobei die mehreren Anordnungen von Klebepunkten (27, 33) so angeordnet sind, dass entsprechende Paare der einzelnen Klebepunkte in den mehreren Anordnungen, die in der Querrichtung (X) benachbart sind, sich niemals übereinander legen.

## Revendications

1. Procédé pour fabriquer un article à porter comprenant un châssis (2) et des fixations mécaniques (3) en tant que composants de base, ledit châssis (2) ayant une direction longitudinale (Y), une direction transversale (X), un côté (4) orienté vers la peau de l'utilisateur et un côté (5) orienté vers les vêtements de l'utilisateur, une première région de ceinture (8) correspondant à l'une des régions de ceinture avant et arrière, une seconde région de ceinture (9) correspondant à l'autre parmi lesdites régions de ceinture avant et arrière et une région d'entre-jambes (10) s'étendant entre lesdites première et seconde régions de ceinture (8, 9), et lesdites fixations mécaniques (3) comprennent des premiers éléments de fixation mécanique (15, 16) prévus le long d'une paire de premières zones latérales (11) de ladite première région de ceinture (8) opposées entre elles dans ladite direction transversale (X) afin de s'étendre dans la direction longitudinale (Y) et des seconds éléments de fixation mécanique (17, 18) prévus le long d'une paire de secondes zones latérales (12) de ladite seconde région de ceinture (9) opposées entre elles dans ladite direction transversale (X) afin de s'étendre dans ladite direction longitudinale (Y), dans lequel lesdits premiers éléments de fixation mécanique (15, 16) sont mis en prise de manière détachable avec lesdits seconds éléments de fixation mécanique (17, 18) et dans lequel lesdits premiers éléments de fixation mécanique (15, 16) sont formés le long desdites premières zones latérales (11) par l'intermédiaire des éléments de montage (13, 14), ledit procédé comprenant les étapes consistant à :
replier chacun desdits éléments de montage (13, 14) le long d'une première ligne de pliage (35) s'étendant dans ladite direction longitudinale (Y) afin de définir une première région (26, 27) et une deuxième région (28, 29), puis le long d'une seconde ligne de pliage (36) s'étendant également dans ladite direction longitudinale (Y) pour définir ladite deuxième région (28, 29) et une troisième région (30, 31) de sorte que ladite deuxième région (28, 29) peut chevaucher sur ladite première région (26, 27) alors que ladite troisième région (30, 31) peut chevaucher sur ladite deuxième région (28, 29) ;
former un groupe de points adhésifs (32, 33) entre ladite deuxième région (28, 29) et ladite troisième région (30, 31) pour fixer temporairement ladite deuxième région (28, 29) sur ladite troisième région (30, 31) ;
relier ladite troisième région (30, 31) audit premier élément de fixation mécanique (15, 16) associé ;
mettre en prise ledit premier élément de fixation mécanique (15, 16) avec ledit second élément de fixation mécanique (17, 18) associé ;
relier ladite première région (26, 27) auxdites premières zones latérales (11) associées ; et
relier ledit second élément de fixation mécanique (17, 18) à ladite seconde zone latérale (12) associée ;
**caractérisé en ce que** les premiers éléments de fixation mécanique sont respectivement divisés en moitiés supérieures (15a, 16a) et moitiés inférieures (15b, 16b) dans la direction longitudinale (Y) afin de former des espaces entre eux ; les groupes de points adhésifs (32, 33) sont formés sensiblement sur toute une longueur dudit élément de montage (13, 14) dans ladite direction longitudinale (Y) ; et les groupes de points adhésifs (32, 33) sont formés dans des régions chevauchant les moitiés supérieures et inférieures (15a, 15b ; 16a, 16b) des premiers éléments de fixation mécanique et dans des régions chevauchant lesdits espaces.

2. Procédé pour fabriquer un article à porter comprenant un châssis (2) et des fixations mécaniques (3) en tant que composants de base, ledit châssis (2) ayant une direction longitudinale (Y), une direction transversale (X), un côté (4) orienté vers la peau de l'utilisateur, et un côté (5) orienté vers les vêtements de l'utilisateur, une première région de ceinture (8) correspondant à l'une parmi les régions de ceinture avant et arrière, une seconde région de ceinture (9) correspondant à l'autre parmi lesdites régions de ceinture avant et arrière et une région d'entre-jambes (10) s'étendant entre lesdites première et seconde régions de ceinture (8, 9), et lesdites fixations mécaniques (3) comprennent des premiers éléments de fixation mécanique (15, 16) prévus le long d'une paire de premières zones latérales (11) de ladite région de ceinture (8) opposées entre elles dans ladite direction transversale (X) afin de s'étendre dans ladite direction longitudinale (Y) et des seconds éléments de fixation mécanique (17, 18) prévus le long d'une paire de secondes zones latérales (12) de ladite seconde région de ceinture (9) opposées entre elles dans ladite direction transversale (X) afin de s'étendre dans ladite direction longitudinale (Y), dans lequel lesdits premiers éléments de fixation mécanique (15, 16) sont mis en prise de manière détachable avec lesdits seconds éléments de fixation mécanique (17, 18) et dans lequel lesdits premiers éléments de fixation mécanique (15, 16) sont formés le long desdites premières zones latérales (11) par l'intermédiaire des éléments de montage (13, 14), ledit procédé comprenant les étapes consistant à :
replier chacun desdits éléments de montage (13, 14) le long d'une première ligne de pliage (35) s'étendant dans ladite direction longitudinale (Y) pour définir une première région (26, 27) et une deuxième région (28, 29), puis le long d'une seconde ligne de pliage (36) s'étendant également dans ladite direction longitudinale (Y) afin de définir ladite deuxième région (28, 29) et une troisième région (30, 31) de sorte que ladite deuxième région (28, 29) peut chevaucher sur ladite première région (26, 27) alors que ladite troisième région (30, 31) peut chevaucher sur ladite deuxième région (28, 29) ;
former un groupe de points adhésifs (32, 33) entre ladite deuxième région (28, 29) et ladite troisième région (30, 31) pour fixer temporairement ladite deuxième région (28, 29) à ladite troisième région (30, 31) ;
mettre en prise ledit premier élément de fixation mécanique (15, 16) avec ledit second élément de fixation mécanique (17, 18) associé ;
relier ladite troisième région (30, 31) audit premier élément de fixation mécanique (15, 16) associé mis en prise avec ledit second élément de fixation mécanique (17, 18) ;
relier ladite première région (26, 27) à ladite première zone latérale (11) associée ; et
relier ledit second élément de fixation mécanique (17, 18) à ladite seconde zone latérale (12) associée ;
**caractérisé en ce que** les premiers éléments de fixation mécanique sont respectivement divisés en moitiés supérieures (15a, 16a) et moitiés inférieures (15b, 16b) dans la direction longitudinale (Y) afin de former des espaces entre eux ; les groupes de points adhésifs (32, 33) sont sensiblement formés sur toute une longueur dudit élément de montage (13, 14) dans ladite direction longitudinale (Y) ; et les groupes de points adhésifs (32, 33) sont formés dans des régions chevauchant les moitiés supérieures et inférieures (15a, 15b ; 16a, 16b) des premiers éléments de fixation mécanique et dans des régions chevauchant lesdits espaces.

3. Procédé pour fabriquer l'article à porter selon la revendication 1 ou 2, dans lequel un procédé pour fabriquer ledit article à porter a un sens machine (MD) et un sens travers (CD) orthogonal audit sens machine (MD) et le châssis (2) dudit article à porter est formé en continu de sorte que ladite direction transversale (X) dudit châssis (2) correspond audit sens machine (MD), ledit procédé comprend en outre les étapes consistant à :
amener la bande (41, 42) formant ledit châssis (2) dans ledit sens machine (MD) ; et
couper ledit châssis (2) dans ledit sens travers (CD) après que ledit premier élément de fixation mécanique (15, 16) a été mis en prise avec ledit second élément de fixation mécanique (17, 18) associé ;
dans lequel lesdites premières zones latérales (11) sont formées le long d'une extrémité de ladite bande (41, 42) s'étendant dans le sens machine (MD) et lesdites secondes zones latérales (12) sont formées le long de l'extrémité opposée.

4. Procédé pour fabriquer l'article à porter selon la revendication 3, dans lequel des paires respectives desdits éléments de montage (13, 14) adjacents desdits articles à porter formées en continu dans ledit sens machine (MD) sont contiguës entre elles dans lesdites troisièmes régions (30, 31) respectives et coupées dans ces troisièmes régions (30, 31) contiguës dans ledit sens travers (CD).

5. Article à porter comprenant un châssis (2) et des fixations mécaniques (3) en tant que composants de base, ledit châssis (2) ayant une direction longitudinale (Y), une direction transversale (X), un côté (4) orienté vers la peau de l'utilisateur et un côté (5) orienté vers les vêtements de l'utilisateur, une première région de ceinture (8) correspondant à l'une parmi des régions de ceinture avant et arrière, une seconde région de ceinture (9) correspondant à l'autre parmi lesdites régions de ceinture avant et arrière et une région d'entre-jambes (10) s'étendant entre lesdites première et seconde régions de ceinture (8, 9), et lesdites fixations mécaniques (3) comprennent des premiers éléments de fixation mécanique (15, 16) prévus le long d'une paire de premières zones latérales (11) de ladite première région de ceinture (8) opposées entre elles dans ladite direction transversale (X) afin de s'étendre dans la direction longitudinale (Y) et des seconds éléments de fixation mécanique (17, 18) prévus le long d'une paire de secondes zones latérales (12) de ladite seconde région de ceinture (9) opposées entre elles dans ladite direction transversale (X) afin de s'étendre dans ladite direction longitudinale (Y), dans lequel lesdits premiers éléments de fixation mécanique (15, 16) sont mis en prise de manière détachable avec lesdits seconds éléments de fixation mécanique (17, 18) et dans lequel lesdits premiers éléments de fixation mécanique (15, 16) sont formés le long desdites premières zones latérales (11) par l'intermédiaire des éléments de montage (13, 14), ledit article à porter comprenant en outre :
chacun desdits éléments de montage (13, 14) comprend une première ligne de pliage (35), une seconde ligne de pliage (36), une première région (26, 27) et une deuxième région (28, 29) définies par ladite première ligne de pliage (35) et ladite seconde ligne de pliage (36), et une troisième région (30, 31) définie conjointement avec ladite deuxième région (28, 29) par ladite seconde ligne de pliage, dans lequel ladite deuxième région (28, 29) chevauche sur ladite première région (26, 27) et ladite troisième région (30, 31) chevauche sur ladite deuxième région (28, 29) ;
ladite troisième région (30, 31) est reliée audit premier élément de fixation mécanique (15, 16) associé ;
ladite première région (26, 27) est reliée à ladite première zone latérale (11) associée ; et
un groupe de points adhésifs (32, 33) est formé entre ladite deuxième région (28, 29) et ladite troisième région (30, 31) pour fixer temporairement ladite deuxième région (28, 29) à ladite troisième région (30, 31) ;
**caractérisé en ce que** les premiers éléments de fixation mécanique sont respectivement divisés en moitiés supérieures (15a, 16a) et moitiés inférieures (15b, 16b) dans la direction longitudinale (Y) afin de former des espaces entre eux ; les groupes de points adhésifs (32, 33) sont sensiblement formés sur toute une longueur dudit élément de montage (13, 14) dans ladite direction longitudinale (Y) ; et les groupes de points adhésifs (32, 33) sont formés dans des régions chevauchant les moitiés supérieures et inférieures (15a, 15b ; 16a, 16b) des premiers éléments de fixation mécanique et dans des régions chevauchant lesdits espaces.

6. Article à porter selon la revendication 5, dans lequel ledit groupe de points adhésifs (32, 33) est formé afin de chevaucher sur une région dans laquelle ledit premier élément de fixation mécanique (15, 16) est mis en prise avec ledit second élément de fixation mécanique (17, 18).

7. Article à porter selon la revendication 5 ou 6, dans lequel ledit groupe de points adhésifs (32, 33) est formé au moins le long de ladite extrémité transversalement interne de ladite région dans laquelle ledit premier élément de fixation mécanique (15, 16) est mis en prise avec ledit second élément de fixation mécanique (17, 18) associé.

8. Article à porter selon l'une quelconque des revendications 5 à 7, dans lequel ledit groupe de points adhésifs (32, 33) est formé de manière intermittente sensiblement sur toute une longueur dudit élément de montage (13, 14) dans ladite direction longitudinale (Y).

9. Article à porter selon l'une quelconque des revendications 5 à 8, dans lequel une pluralité desdits groupes de points adhésifs (32, 33) sont formés afin d'être espacés les uns des autres dans ladite direction transversale (X).

10. Article à porter selon la revendication 9, dans lequel ladite pluralité de groupes de points adhésifs (27, 33) sont agencés de sorte que des paires respectives de points adhésifs individuels dans ladite pluralité de groupes qui sont adjacents dans ladite direction transversale (X) ne se chevauchent jamais.
